# EUROPÄISCHE PATENTSCHRIFT

(11) **EP 1 996 371 B1**
(45) Veröffentlichungstag und Bekanntmachung des Hinweises auf die Patenterteilung: **18.11.2009**
(21) Anmeldenummer: 07722044.0
(22) Anmeldetag: 14.03.2007
(51) Int. Cl.: B25B 23/143, F16D 7/10, F16D 43/208, A61B 17/88

(54) **DREHMOMENTBEGRENZER**
TORQUE LIMITER
LIMITEUR DE COUPLE

(30) Priorität: 15.03.2006 DE 202006004027 U
(43) Veröffentlichungstag der Anmeldung: 03.12.2008
(73) Patentinhaber: stryker Trauma GmbH, 24232 Schönkirchen/Kiel (DE)
(72) Erfinder: Witte, Peter, 24114 Kiel (DE)
(74) Vertreter: Biehl, Christian
(86) Internationale Anmeldenummer: PCT/DE2007/000471
(87) Internationale Veröffentlichungsnummer: WO 2007/104296

(56) Entgegenhaltungen:
- DE-A1- 19 722 798
- DE-A1- 19 903 863
- GB-A- 894 306
- US-A- 2 668 426
- US-A- 2 960 852
- US-A- 3 067 597

## Beschreibung

Die Erfindung betrifft einen Drehmomentbegrenzer, insbesondere einen Drehmomentbegrenzer, der zum Begrenzen des Drehmoments eines chirurgischen Schraubendrehers geeignet ist.

Ein derartiger Drehmomentbegrenzer für einen chirurgischen Schraubendreher ist aus der DE 600 04 376 T2 bekannt. Dieser Drehmomentbegrenzer ist mechanisch aufwendig.

Weiter sind die DE 197 22 798 A1, die US-A-2 668 426 und die GB 894 306 A zu nennen, die Kupplungen zur Begrenzung des Drehmoments beschreiben, bei denen jedoch eine Auskupplung nicht drehrichtungsspezifisch ist und die nicht für den Einsatz eines Schraubendrehers eingerichtet sind.

Der Erfindung liegt die Aufgabe zu Grunde, einen Drehmomentbegrenzer zum Begrenzen des Drehmoments eines chirurgischen Schraubendrehers zu schaffen, der mechanisch wenig aufwendig ist.

Erfindungsgemäß wird diese Aufgabe gelöst durch eine Außenhülse, deren Innenwand mit wenigstens einer umlaufenden Nut und einer Mehrzahl von achsparallel verlaufenden Vertiefungen versehen ist, (einen) in die Nut(en) eingesetzten elastischen Ring(en), einen von der Außenhülse aufgenommenen, mit einer der Anzahl der Vertiefungen entsprechenden Anzahl von Wälzkörperaufnahmen versehenen Wälzkörperkäfig, einer Mehrzahl von in die Wälzkörperaufnahmen eingesetzten Wälzkörpern, einer von dem Wälzkörperkäfig aufgenommenen Innenhülse, deren Außenwand mit einer der Anzahl der Wälzkörperaufnahmen entsprechenden Anzahl von sich achsparallel erstreckenden V-förmigen Kerben versehen ist, und Mittel zum Begrenzen der Relativdrehung zwischen Wälzkörperkäfig und Außenhülse zwischen einer ersten, bei Drehen der Außenhülse in Andrehrichtung eingenommenen Position, in der die Mittel eine Übertragung des Drehmoments bewirken und in der die Wälzkörper mit den Vertiefungen fluchten und bei Erreichen des maximal zu übertragenden Drehmoments aus den Kerben in der Innenhülse aus- und unter Verdrängung des (der) elastischen Rings (Ringe) in die Vertiefungen in der Außenhülse eintreten und einer zweiten, bei Drehen der Außenhülse in Löserichtung eingenommenen Position, in der die von den Kerben in der Innenhülse aufgenommenen Wälzkörper in einer gegenüber den Vertiefungen in der Außenhülse versetzten Position sind.

Eine alternative Ausführungsform zeichnet sich aus durch eine Innenhülse, deren Außenwand mit wenigstens einer umlaufenden Nut und einer Mehrzahl von achsparallel verlaufenden Vertiefungen versehen ist, in die Nut(en) eingesetzten elastischen Ring(en), einem die Innenhülse umgebenden, mit einer der Anzahl der Vertiefungen entsprechenden Anzahl von Wälzkörperaufnahmen versehenen Wälzkörperkäfig, eine Mehrzahl von in die Wälzkörperaufnahmen eingesetzten Wälzkörpern, eine den Wälzkörperkäfig umgebende Außenhülse, deren Innenwand mit einer der Anzahl der Wälzkörperaufnahmen entsprechenden Anzahl von sich achsparallel erstreckenden V-förmigen Kerben versehen ist, und Mittel zum Begrenzen der Relativdrehung zwischen Wälzkörperkäfig und Innenhülse zwischen einer ersten, bei Drehen der Außenhülse in Andrehrichtung eingenommenen Position, in der die in den Kerben in der Außenhülse liegenden Wälzkörper mit den Vertiefungen fluchten und bei Erreichen des maximal zu übertragenden Drehmoments aus den Kerben aus- und unter Verdrängung des (der) elastischen Rings (Ringe) in die Vertiefungen eintreten, und einer zweiten, bei Drehen der Außenhülse in Löserichtung eingenommenen Position, in der die von den Kerben aufgenommenen Wälzkörper gegenüber den Vertiefungen versetzten Position sind.

Die Mittel können durch an dem Wälzkörperkäfig gegenüber den Wälzkörperaufnahmen versetzt ausgebildete, in die achsparallel verlaufenden Vertiefungen eingreifende Nasen gebildet werden.

Die Wälzkörper können Nadeln oder Zylinder sein.

Der (die) elastischen Ring(e) ist (sind) vorzugsweise ein aus einem elastischen Material gefertigte(r) O-Ring(e).

Die Erfindung wird im Folgenden anhand einer Zeichnung erläutert. Dabei zeigt:

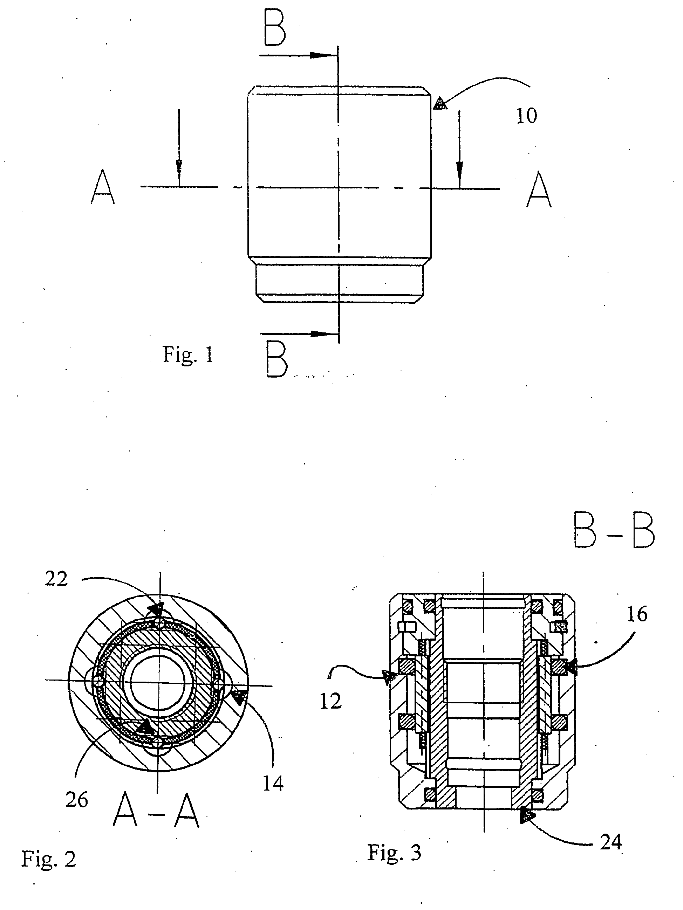

- Fig. 1: eine Ansicht des Drehmomentbegrenzers,
- Fig. 2: eine Schnittansicht entlang der Linie A-A von Fig. 1,
- Fig. 3: eine Schnittansicht entlang der Linie B-B von Fig. 1,

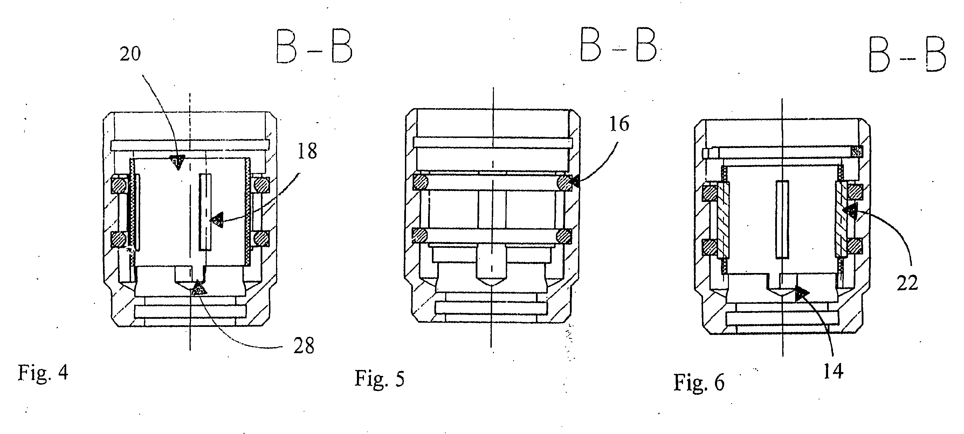

- Fig. 4: eine Schnittansicht entlang der Linie B-B von Fig. 1 ohne In- nenhülse in der zweiten Position, der Löseposition,
- Fig. 5: eine Schnittansicht entlang der Linie B-B von Fig. 1 ohne In- nenhülse und ohne Wälzkörperkäfig und
- Fig. 6: eine Fig. 4 entsprechende Ansicht in der ersten Position, der An- drehposition.

Der Drehmomentbegrenzer besteht aus einer Außenhülse 10, über die eine Rotationskraft auf den Drehmomentbegrenzer aufgebracht wird. Die Innenwand der Außenhülse 10 ist mit wenigstens einer umlaufenden Nut 12 und einer Mehrzahl von achsparallel, also rechtwinklig zu der Nut verlaufenden Vertiefungen versehen. In die Nuten 12 ist jeweils ein elastischer O-Ring 16 eingesetzt.

Die Außenhülse nimmt einen Wälzkörperkäfig 20 auf, der mit einer der Anzahl der Vertiefungen 14 entsprechenden Anzahl von Wälzkörperaufnahmen 18 versehen ist. Jede der Wälzkörperaufuahmen 18 nimmt einen Wälzkörper 22 auf, die radial beweglich sind.

Der Wälzkörperkäfig 20 ist mit einer Anzahl von sich achsparallel erstreckenden Nasen 28 versehen, die in die achsparallel verlaufenden Vertiefungen 14 in der Außenhülse 10 eingreifen, wobei die Nasen 28 schmaler sind als die Breite der Vertiefungen 14, so dass der Wälzkörperkäfig 20 um einige Winkelgrad in der Außenhülse drehbar ist, bis die Seitenfläche der Nasen 28 an der einen Begrenzung oder an der anderen Begrenzung der Vertiefungen 14 anstoßen.

Der Wälzkörperkäfig 20 wiederum nimmt eine Innenhülse 24 auf, in die der (nicht dargestellte) Schaft eines Schraubendrehers eingesetzt ist. Die Außenwand der Innenhülse 24 ist mit V-förmigen Kerben 26, in die die Wälzkörper 22 eintreten können, versehen.

In einer ersten, bei Drehen der Außenhülse 10 in der Andrehrichtung eingenommenen Position fluchten die Wälzkörper 22 mit den Vertiefungen 14. Die an der einen der Wandungen der Vertiefungen anliegenden Nasen 28 übertragen das Drehmoment von der Außenhülse 10 auf den Wälzkörperkäfig 20. Bei Erreichen des maximal zu übertragenden Drehmoments treten die Wälzkörper 22 aus den V-förmigen Kerben 26 in der Innenhülse 24 aus und treten unter Eindringen in die elastischen Ringe 16 in die Vertiefungen 14 in der Außenhülse 10 ein. In dieser Position erfolgt kleine Kraftübertragung zwischen der den Wälzkörperkäfig 20 mitführenden Außenhülse 10 und der Innenhülse 24, ein weiteres Andrehen ist damit nicht möglich.

In einer zweiten, bei Drehen der Außenhülse in Löserichtung eingenommene Position sind die von den Kerben 26 in der Innenhülse 24 aufgenommenen Wälzkörper 22 gegenüber den Vertiefungen der Außenhülse 10 versetzt. Die Wälzkörper sind von den O-Ringen 16 wieder in die Kerben 26 eingedrückt. Die jetzt an der anderen Wandung der Vertiefung anliegenden Nasen 24 bewirken eine Übertragung des Drehmoments von der Außenhülse auf den Wälzkörperkäfig und damit zwischen Außenhülse und. Innenhülse.

Das maximale Drehmoment ist durch Auswahl der O-ringe mit der gewünschten Shore-Härte oder der Anzahl der eingesetzten Wälzkörper bestimmt.

Nicht dargestellt ist eine alternativen, den Gegenstand von Anspruch 2 bildende Ausführungsform, bei der die Vertiefungen und der elastische Ring in der Innenhülse und die Kerben in der Außenhülse angeordnet sind.

## Patentansprüche

1. Drehmomentbegrenzer, insbesondere für einen chirurgischen Schraubendreher, **gekennzeichnet durch**
- eine Außenhülse (10), deren Innenwand mit wenigstens einer umlaufenden Nut (12) und einer Mehrzahl von achsparallel verlaufenden Vertiefungen (14) versehen ist,
- jeweils einen in die wenigstens eine Nut (12) eingesetzten elastischen Ring (16),
- einen von der Außenhülse (10) aufgenommenen, mit einer der Anzahl der Vertiefungen (14) entsprechenden Anzahl von Wälzkörperaufnahmen (18) versehenen Wälzkörperkäfig (20),
- eine Mehrzahl von in die Wälzkörperaufnahmen (18) eingesetzten Wälzkörpern (22),
- eine von dem Wälzkörperkäfig (20) aufgenommenen Innenhülse (24), deren Außenwand mit einer der Anzahl der Wälzkörperaufnahmen (18) entsprechenden Anzahl von sich achsparallel erstreckenden V-förmigen Kerben (26) versehen ist, und
- Mittel zum Begrenzen der Relativdrehung zwischen Wälzkörperkäfig (20) und Außenhülse (10) zwischen einer ersten, bei Drehen der Außenhülse (10) in Andrehrichtung eingenommenen Position, in der die Mittel eine Übertragung des Drehmoments bewirken und in der die Längsachsen der Wälzkörper (22) mit den Längsachsen der Vertiefungen (14) in radialer Richtung fluchten und bei Erreichen des maximal zu übertragenden Drehmoments aus den Kerben (26) in der Innenhülse (24) aus- und unter Verdrängung des wenigstens einen elastischen Rings (16) in die Vertiefungen (14) in der Außenhülse (10) eintreten und einer zweiten, bei Drehen der Außenhülse (10) in Löserichtung eingenommenen Position, in der die von den Kerben (26) in der Innenhülse (24) aufgenommenen Wälzkörper (22) mit deren Längsachsen in einer gegenüber den Längsachsen der Vertiefungen (14) in der Außenhülse (10) tangential versetzten Position sind.

2. Drehmomentbegrenzer, insbesondere für einen chirurgischen Schraubendreher, **gekennzeichnet durch**
- eine Innenhülse, deren Außenwand mit wenigstens einer umlaufenden Nut und einer Mehrzahl von achsparallel verlaufenden Vertiefungen versehen ist,
- jeweils einen in die wenigstens eine Nut eingesetzten elastischen Ring,
- einem die Innenhülse umgebenden, mit einer der Anzahl der Vertiefungen entsprechenden Anzahl von Wälzkörperaufnahmen versehenen Wälzkörperkäfig,
- eine Mehrzahl von in die Wälzkörperaufnahmen eingesetzten Wälzkörpern,
- eine den Wälzkörperkäfig umgebende Außenhülse, deren Innenwand mit einer der Anzahl der Wälzkörperaufnahmen entsprechenden Anzahl von sich achsparallel erstreckenden V-förmigen Kerben versehen ist, und
- Mittel zum Begrenzen der Relativdrehung zwischen Wälzkörperkäfig und Innenhülse zwischen einer ersten, bei Drehen der Außenhülse in Andrehrichtung eingenommenen Position, in der die Längsachsen der in den Kerben in der Außenhülse liegenden Wälzkörper mit den Längsachsen der Vertiefungen in radialer Richtung fluchten und bei Erreichen des maximal zu übertragenden Drehmoments aus den Kerben aus- und unter Verdrängung des wenigstens einen elastischen Rings in die Vertiefungen eintreten, und einer zweiten, bei Drehen der Außenhülse in Löserichtung eingenommenen Position, in der die von den Kerben aufgenommenen Wälzkörper mit deren Längsachsen in einer gegenüber den Längsachsen der Vertiefungen tangential versetzten Position sind.

3. Drehmomentbegrenzer nach Anspruch 1 oder 2, **dadurch gekennzeichnet, dass** die Mittel durch an dem Wälzkörperkäfig (20) gegenüber den Wälzkörperaufnahmen (18) versetzt ausgebildete, in die achsparallel verlaufenden Vertiefungen (14) eingreifende Nasen (28) gebildet werden.

4. Drehmomentbegrenzer nach Anspruch 1, 2 oder 3, **dadurch gekennzeichnet, dass** die Wälzkörper (22) Nadeln sind.

5. Drehmomentbegrenzer nach einem der vorangehenden Ansprüche, **dadurch gekennzeichnet, dass** der elastische Ring (16) ein aus einem elastischen Material gefertigter O-Ring ist.

## Claims

1. A torque limiter, in particular for a surgical screwdriver, **characterized by**
- an outside sleeve (10) whose inside wall is provided with at least one circumferential groove (12) and a plurality of depressions (14) that run axially parallel,
- in each case an elastic ring (16) inserted into the at least one groove (12),
- a rolling element cage (20) received by the outside sleeve (10) and provided with a number of rolling element recesses (18) corresponding to the number of depressions (14),
- a plurality of rolling elements (22) inserted into the rolling element recesses (18),
- an inside sleeve (24) that is received by the rolling element cage (20) and whose outside wall is provided with V-shaped notches (26) that extend axially parallel and whose number corresponds to the number of the rolling element recesses (18), and
- means for limiting the relative rotation between rolling element cage (20) and outside sleeve (10) between a first position that is assumed when rotating the outside sleeve (10) in the tightening direction, in which position the means effect a transmission of the torque and in which the longitudinal axes of the rolling elements (22) are flush with the longitudinal axes of the depressions (14) in the radial direction and exit the notches (26) in the inside sleeve (24) on reaching the maximum torque to be transmitted and enter the depressions (14) in the outside sleeve (10) while displacing the at least one elastic ring (16), and a second position that is assumed when the outside sleeve (10) is rotated in the releasing direction, in which position the rolling elements (22) that are received by the notches (26) in the inside sleeve (24) are with their longitudinal axes in a position that is displaced tangentially relative to the longitudinal axes of the depressions (24) in the outside sleeve (10).

2. The torque limiter, in particular for a surgical screwdriver, **characterized by**
- an inside sleeve whose outside wall is provided with at least one circumferential groove and a plurality of depressions that run axially parallel,
- in each case an elastic ring inserted into the at least one groove,
- a rolling element cage surrounding the inside sleeve and provided with a number of rolling element recesses corresponding to the number of depressions,
- a plurality of rolling elements inserted into the rolling element recesses,
- an outside sleeve that surrounds the rolling element cage and whose inside wall is provided with V-shaped notches that extend axially parallel and whose number corresponds to the number of the rolling element recesses, and
- means for limiting the relative rotation between rolling element cage and inside sleeve between a first position that is assumed when rotating the outside sleeve in the tightening direction, in which position the longitudinal axes of the rolling elements lying in the notches in the outside sleeve are flush with the longitudinal axes of the depressions in the radial direction and exit the notches on reaching the maximum torque to be transmitted and enter the depressions while displacing the at least one elastic ring, and a second position that is assumed when the outside sleeve is rotated in the releasing direction, in which position the rolling elements that are received by the notches are with their longitudinal axes in a position that is displaced tangentially relative to the longitudinal axes of the depressions.

3. The torque limiter according to Claim 1 or 2, **characterized in that** the means are formed by lugs (28) that engage in depressions (14) that run axially parallel and are formed offset relative to the rolling element recesses (18).

4. The torque limiter according to Claim 1, 2 or 3, **characterized in that** the rolling elements (22) are needles.

5. The torque limiter according one of the preceding claims, **characterized in that** the elastic ring (16) is an O ring made from an elastic material.

## Revendications

1. Limiteur de couple, en particulier pour un tournevis chirurgical, **caractérisé par**
- une douille extérieure (10) dont la paroi interne est pourvue d'au moins une rainure périphérique (12) et d'une pluralité de creux (14) s'étendant parallèlement à l'axe,
- respectivement une bague élastique (16) mise en place dans ladite au moins une rainure (12),
- une cage à éléments roulants (20) qui est reçue par ladite douille extérieure (10) et qui est pourvue d'un nombre de logements d'éléments roulants (18) qui correspond au nombre des creux (14),
- une pluralité d'éléments roulants (22) insérés dans lesdits logements d'éléments roulants (18),
- une douille intérieure (24) qui est reçue par ladite cage à éléments roulants (20) et dont la paroi externe est pourvue d'un nombre d'entailles en V (26) s'étendant parallèlement à l'axe, ce nombre correspondant au nombre des logements d'éléments roulants (18), et
- des moyens destinés à limiter la rotation relative entre la cage à éléments roulants (20) et ladite douille extérieure (10), entre une première position prise en tournant la douille extérieure (10) dans la direction de serrage, dans laquelle les moyens provoquent une transmission du couple et les axes longitudinaux des éléments roulants (22) sont alignés dans la direction radiale avec les axes longitudinaux des creux (14) et dans laquelle, lorsque le couple à transmettre au maximum est atteint, les éléments roulants quittent lesdites entailles (26) réalisées dans la douille intérieure (24) et entrent, en déplaçant ladite au moins une bague élastique (16), dans les creux (14) ménagés dans la douille extérieure (10), et une deuxième position prise en tournant la douille extérieure (10) dans la direction de desserrage, dans laquelle les éléments roulants (22) reçus par les entailles (26) réalisées dans la douille intérieure (24) se trouvent, avec les axes longitudinaux de celles-ci, dans une position décalée tangentiellement par rapport aux axes longitudinaux des creux (14) ménagés dans la douille extérieure (10).

2. Limiteur de couple, en particulier pour un tournevis chirurgical, **caractérisé par**
- une douille intérieure dont la paroi externe est pourvue d'au moins une rainure périphérique ainsi que d'une pluralité de creux s'étendant parallèlement à l'axe,
- respectivement une bague élastique mise en place dans ladite au moins une rainure,
- une cage à éléments roulants entourant ladite douille intérieure et pourvue d'un nombre de logements d'éléments roulants qui correspond au nombre des creux,
- une pluralité d'éléments roulants insérés dans lesdits logements d'éléments roulants,
- une douille extérieure qui entoure ladite cage à éléments roulants et dont la paroi interne est pourvue d'un nombre d'entailles en V s'étendant parallèlement à l'axe, ce nombre correspondant au nombre des logements d'éléments roulants, et
- des moyens destinés à limiter la rotation relative entre la cage à éléments roulants et la douille intérieure, entre une première position prise en tournant la douille extérieure dans la direction de serrage, dans laquelle les axes longitudinaux des éléments roulants situés dans les entailles réalisées dans la douille extérieure sont alignés dans la direction radiale avec les axes longitudinaux des creux et dans laquelle, lorsque le couple à transmettre au maximum est atteint, les éléments roulants quittent les entailles et entrent, en déplaçant ladite au moins une bague élastique, dans les creux, et une deuxième position prise en tournant la douille extérieure dans la direction de desserrage, dans laquelle les éléments roulants reçus par les entailles se trouvent, avec les axes longitudinaux de celles-ci, dans une position décalée tangentiellement par rapport aux axes longitudinaux des creux.

3. Limiteur de couple selon la revendication 1 ou 2, **caractérisé par le fait que** lesdits moyens sont formés par des nez (28) qui sont réalisés sur la cage à éléments roulants (20) de manière à être décalés par rapport aux logements d'éléments roulants (18) et qui s'engagent dans les creux (14) s'étendant parallèlement à l'axe.

4. Limiteur de couple selon la revendication 1, 2 ou 3, **caractérisé par le fait que** lesdits éléments roulants (22) sont des aiguilles.

5. Limiteur de couple selon l'une quelconque des revendications précédentes, **caractérisé par le fait que** ladite bague élastique (16) est un joint torique réalisé dans une matière élastique.
